(19) Europäisches Patentamt
European Patent Office
Office européen des brevets

(11) **EP 2 700 427 B1**

(12) **EUROPÄISCHE PATENTSCHRIFT**

(45) Veröffentlichungstag und Bekanntmachung des Hinweises auf die Patenterteilung:
**26.11.2014 Patentblatt 2014/48**

(51) Int Cl.:
*A61M 5/44* (2006.01)         *A61N 1/08* (2006.01)
*H05B 3/26* (2006.01)         *F16L 53/00* (2006.01)

(21) Anmeldenummer: **12180968.5**

(22) Anmeldetag: **20.08.2012**

(54) **Fluidwärmer und Verfahren zum Betrieb eines Fluidwärmers**

Fluid warmer and method for operating same

Dispositif de chauffage de fluide et procédé de fonctionnement d'un dispositif de chauffage de fluide

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.02.2014 Patentblatt 2014/09**

(73) Patentinhaber: **WWT Technischer Gerätebau GmbH**
**70190 Stuttgart (DE)**

(72) Erfinder:
• **Theilacker-Beck, Wolfgang**
**70176 Stuttgart (DE)**

• **Theilacker, Matthias**
**70563 Stuttgart (DE)**
• **Schmider, Klaus H.**
**70180 Stuttgart (DE)**

(74) Vertreter: **Kohler Schmid Möbus**
**Patentanwälte**
**Ruppmannstraße 27**
**70565 Stuttgart (DE)**

(56) Entgegenhaltungen:
**EP-A2- 0 504 835         WO-A2-2009/105413**
**DE-A1-102010 036 295     GB-A- 2 052 109**

EP 2 700 427 B1

**Beschreibung**

Die Erfindung betrifft einen Fluidwärmer und ein Verfahren zum Betrieb eines Fluidwärmers.

Die Applikation größerer Mengen ungewärmter Fluide, beispielsweise beim intravenösen Verabreichen von Blutprodukten oder Infusionslösungen, kann nicht zuletzt aufgrund des damit einhergehenden Verlusts an Körperwärme nachteilige physiologische Effekte haben.

In der klinischen Praxis werden daher routinemäßig Fluidwärmer eingesetzt, um das in der Fluidleitung strömend geführte medizinische Fluid auf eine Temperatur nahe der Körpertemperatur von üblicher Weise 37° Celsius vorzuwärmen.

Die Fluidwärmer weisen nach einer am Markt etablierten Bauart ein oder mehrere Widerstandsheizelemente für Wechselspannung auf. Die Widerstandsheizelemente sind mit einem ersten und einem zweiten elektrischen Netzanschlussleiter versehen, um das Widerstandsheizelement mit einer Wechselspannungsquelle elektrisch leitend zu verbinden. Ein Wärmeübertrager, der mit dem Widerstandsheizelement thermisch leitend verbunden ist, dient einer Wärmeübertragung auf ein mit der Fluidleitung verbundenes oder durch diese ausgebildetes beutelförmiges Behältnis und das darin geführte Fluid. Der Wärmeübertrager ist häufig plattenförmig ausgebildet, um eine effiziente Wärmeübertragung auf das beutelförmige Behältnis zu gewährleisten.

Die temperierten Fluide kommen in der Praxis typischer Weise unter Umgehung des Hautwiderstands mit der Person in einen elektrisch leitenden Kontakt. An Wechselspannung, d.h., insbesondere Netzspannung mit 100 V bis 240 V, betriebene Widerstandsheizelemente können aufgrund einer kapazitiven Kopplung mit anderen Bauteilen des Fluidwärmers, insbesondere dem Wärmeübertragungselement, zu Störungen anderer Diagnostik- bzw. Therapiegeräte, beispielsweise von EKG-Geräten, oder auch von elektrisch erregbaren Organstrukturen, insbesondere des Herzens, führen. Die im medizinischen Bereich eingesetzten Fluidwärmer müssen daher grundsätzlich hohen Anforderungen an die elektrische Sicherheit genügen und die strengen Sicherheitsnormen der international einheitlichen Klassifizierung "CF (cardiac floating)" erfüllen. Hiernach dürfen Patientenableitströme, d.h., über eine mit dem Fluid in Kontakt stehende Person geführte Leckströme, eine Stromstärke von insgesamt 10 µA (Mikroampere) unter Normalbedingungen und 50 µA im Fehlerfall nicht überschreiten.

Aus der DE 10 2010 036 295 A1 ist ein Fluidwärmer bekannt geworden, bei dem der Wärmeübertrager mittels einer einfach oder doppelt ausgeführten Basisisolation von dem Wechselspannungs-Widerstandsheizelement isoliert ist. Die Netzanschlussleiter des Widerstandsheizelements weisen Schalter auf. Zur Überwachung der Qualität der Basisisolation gegenüber dem spannungsführenden Widerstandsheizelement dient ein Isolationswächter. Bei Über- bzw. Unterschreiten eines vorgegebenen Grenzwerts nimmt der Isolationswächter über Schalter eine Abschaltung der Stromversorgung des Widerstandsheizelements vor.

EP 0 504 835 A2 zeigt elektromedizinische Schutzschaltungen zum Schutze eines Patienten, der zur Untersuchung, Therapie oder während einer Operation an elektrischen Geräten angeschlossen ist. Zwischen den zum Körper des Patienten führenden Leitungen sind jeweils spannungsbegrenzende Bauelemente wie z.B. gegenpolig und parallel geschaltete Gleichrichterdioden angeordnet.

Es ist Aufgabe der Erfindung einen eingangs genannten Fluidwärmer mit einem Widerstandsheizelement für Wechselspannung und ein Verfahren zum Betrieb eines solchen Fluidwärmers anzugeben, die ein nochmals höheres Maß an Patientensicherheit gewährleisten.

Die den Fluidwärmer betreffende Aufgabe wird durch einen Fluidwärmer mit den in Patentanspruch 1 angegebenen Merkmalen und die das Verfahren betreffende Aufgabe durch ein Betriebsverfahren mit den in Patentanspruch 16 angegebenen Merkmalen gelöst.

Weitere Vorteile und vorteilhafte Ausgestaltungen des Gegenstands der Erfindung ergeben sich aus der Beschreibung, den Ansprüchen und der Zeichnung.

Bei dem erfindungsgemäßen Fluidwärmer ist der Wärmeübertrager in seiner Funktion als Wärmeübertragungselement im Betrieb funktionsgeerdet. Dadurch kann im Bereich des Wärmeübertragers ein örtlicher Potenzialausgleich geschaffen werden, ohne dass hierzu Ableitströme als sogenannte Patientenableitströme über die Person (oder das Tier) abgeleitet werden, die (das) mit dem in der Fluidleitung und dem Behältnis geführten Fluid in einer elektrisch leitenden Verbindung stehen. Dadurch kann der Gefahr von Herzrhythmusstörungen oder auch Störungen von anderen medizintechnischen Apparaten und Geräten, wie beispielsweise einem EKG-Gerät, zuverlässig entgegengewirkt werden.

Durch das Überführen der Schalter der beiden Netzanschlussleiter und des Funktionserdungsleiters in den geöffneten Schaltzustand bei Detektion eines Ableitstroms, der gleich oder größer als die (jeweils) vorgegebene Schwellenstromstärke ist, kann im stand-alone Betrieb des Fluidwärmers wie auch bei Betrieb im Verbund mit anderen (medizinischen) Geräten eine Patientengefährdung durch zu hohe elektrische Patientenableitströme zuverlässig vermieden werden. Darüber hinaus kann durch das vollständige Trennen der elektrisch leitfähigen Verbindung zwischen dem Widerstandsheizelement und der Wechselspannungsquelle und dem zeitgleichen Unterbrechen einer elektrischen Stromführung über den Funktionserdungsleiter des Wärmeübertragers ein Ableiten von gefahrvollen Fehlerströmen über den Fluidwärmer wirkungsvoll unterbunden werden. Aufgrund der vollständigen elektrischen Trennung des Widerstandsheizelements von der Wechselspannungsquelle wird dabei auch ein möglicher Stromfluss über den mit einem Neutralleiter der Wechselspannungsquelle verbundenen elektrischen Anschlussleiter unterbunden. Dies unabhängig davon, welcher der beiden elektrischen Anschlussleiter jeweils mit einem unter Spannung stehenden Außenleiter (umgangssprachlich auch als *Phase* bezeichnet) bzw. dem (spannungsfreien) Neutralleiter der Wechselspannungsquelle verbunden ist. Das Überführen der Schalter in ihren geöffneten Schaltzustand kann erfindungsgemäß durch eine entsprechend programmierte oder hardwaremäßig konfigurierte Steuereinrichtung realisiert sein. Wesentlich für den Patientenschutz ist ein zügiges Ansprechen der Steuereinrichtung bei Erfassung eines zu hohen Ableitstroms, d.h., ein schnelles Überführen der Schalter in ihren geöffneten Schaltzustand. Unter dem geöffneten Schaltzustand wird derjenige Schaltzustand der Schalter verstanden, bei dem eine Stromleitung über die Schalter unterbrochen ist. Das vorgegebene Zeitintervall, innerhalb dessen die Schalter in ihren geöffneten Schaltzustand überführt werden, ist immer so bemessen, dass eine Gefährdung des Patienten durch Fehler- bzw. Patientenableitstöme sicher ausgeschlossen werden kann.

Der Fluidwärmer weist erfindungsgemäß eine elektrische Kompensationseinrichtung zum Kompensieren des durch den Betrieb des Widerstandsheizelements induzierten Ableitstroms auf. Die Gefahr einer möglichen Patientengefährdung wird dadurch nochmals weiter verringert. Auch kann dadurch die zur Erfassung des Ableitstroms erforderliche Sensorik auf messtechnisch einfachere Weise realisiert werden.

Die Kompensationseinrichtung kann nach der Erfindung insbesondere einen elektrischen LC-Schwingkreis mit einer Spule L und einem Kondensator C aufweisen, der an dem Wärmeübertrager angeschlossen ist. Der Kondensator und die Spule können dabei insbesondere in Reihe geschaltet sein. Die Spule kann im konstruktiv einfachsten Fall insbesondere als Sekundärseite eines Transformators T ausgebildet sein, der primärseitig zum Widerstandsheizelement elektrisch parallel geschaltet ist. D.h., der Transformator ist primärseitig an die Netzanschlussleiter des Widerstandsheizelements angeschlossen und kann somit zeitgleich mit dem Widerstandsheizelement an der Wechselspannungsquelle betrieben werden. Der LC-Schwingkreis kann dadurch zu (erzwungenen) elektrischen Schwingungen mit einer der Wechsel- bzw. Netzspannung entsprechenden Frequenz angeregt werden. Aufgrund der Phasenverschiebung des dadurch verursachten Stroms im LC-Schwingkreis kann der durch den zeitgleichen Betrieb des Widerstandsheizelements induzierte (kapazitive) Ableitstrom des Wärmeübertragers im Wesentlichen kompensiert werden. Der oben beschriebene Schwingkreis kann auch mit elektronischen Bauelementen aufgebaut sein (z.B. unter Verwendung eines Mikroprozessors).

Nach der Erfindung können für einen Betriebszustand des Fluidwärmers mit aktiviertem Widerstandsheizelement und für einen Betriebszustand mit deaktiviertem Widerstandsheizelement unterschiedliche Schwellenströmstärken definiert/vorgegeben sein. Bei einem aktivierten, d.h., von einem von der Wechselspannungsquelle bereitgestellten Heizstrom durchflossenen, Widerstandsheizelement können die messbaren Ableitströme über den Funktionserdungsleiter kleiner sein als bei einem deaktivierten, d.h., einem nicht von einem Heizstrom durchflossenen Widerstandsheizelement. Durch das Vorgeben unterschiedlicher maximaler Schwellenstromstärken $I_{TOn}$, $I_{TOff}$ für die beiden Betriebszustände des Widerstandsheizelements kann diesem Sachverhalt in der Praxis Rechnung getragen werden. Dadurch können unnötige Unterbrechungen des Temperiervorgangs aufgrund einer zu niedrig bemessenen Schwellenstromstärke bei aktiviertem Widerstandsheizelement vermieden und ein effizientes Temperieren der Fluidleitung bzw. des darin geführten medizinischen Fluids gewährleistet werden.

Die maximale Schwellenstromstärke $I_{TOn}$ entspricht vorzugsweise der Summe einer Stromstärke $I_{LOn}$ des Ableitstroms bei einer maximalen Heizleistung des Widerstandsheizelements und einer bevorzugt frei vorgebbaren Differenzstromstärke $\Delta I$. Nach einem Anschalten des Fluidwärmers, beispielsweise vor einem sogenannten Heizmodus, ist die Stromstärke des Ableitstroms über den Funktionserdungsleiter vorzugsweise bei einer maximalen Heizleistung des Widerstandsheizelements bestimmbar. Dies kann beispielsweise im Wege einer entsprechend programmierten oder hardwaremäßig ausgestalteten Steuereinrichtung realisiert werden. Die Differenzstromstärke kann erfindungsgemäß durch eine Bedienperson vorgebbar sein oder ist in der Steuerungseinrichtung (fix) hinterlegt. Einem variablen Leckstromverhatten des Fluidwärmers kann dadurch auf einfache Weise Rechnung getragen werden.

Die Differenzstromstärke $\Delta I$ kann erfindungsgemäß als maximale Schwellenstromstärke $I_{TOff}$ für den Ableitstrom bei einem Betriebszustand des Fluidwärmers mit deaktiviertem Widerstandsheizelement vorgegeben sein.

Nach einer Weiterbildung der Erfindung entspricht die maximale Schwellenstromstärke der Summe der Differenzstromstärke $\Delta I$ und einer Stromstärke $I_{LOff}$ des Ableitstroms bei im geschlossenen Schaltzustand befindlichem Schalter des außenleiterseitig angeschlossenen und damit unter Spannung stehenden Netzanschlussleiters und bei zeitgleich im geöffneten (elektrisch nicht-leitenden) Schaltzustand befindlichem Schalter des neutralleiterseitig mit der Wechselspannungsquelle verbundenen Netzanschlussleiters des Widerstandsheizelements.

Die Differenzstromstärke kann einen Wert zwischen 1 Mikroampere und 50 Mikroampere aufweisen. Die Differenzstromstärke ist bei einem zeitgleichen Einsatz des Fluidwärmers mit einem oder mehreren anderen Geräten am Patienten vorzugsweise kleiner als bei einem stand-alone Einsatz des Fluidwärmers.

Nach einer bevorzugten Weiterbildung der Erfindung sind die Schalter bei einer Stromstärke $I_L$ des Ableitstroms, die kleiner oder gleich einer vorgegebenen minimalen Schwellenstromstärke $I_{LOn}$, $I_{LOff}$ ist, innerhalb des vorgegebenen Zeitintervalls durch die Steuereinrichtung gemeinsam in den geöffneten Schaltzustand überführbar. Dies kann erfindungsgemäß insbesondere durch eine entsprechend programmierte oder hardwaremäßig ausgestaltete Steuereinrichtung realisiert werden. Dadurch kann der Patientenschutz insgesamt nochmals weiter erhöht werden. Im Falle eines stand-alone Betriebs des Fluidwärmers am Patienten kann dadurch eine schwerwiegende Funktionsstörung des Fluidwärmers mit einem unterbrochenen Funktionserdungsleiter erkannt und das Widerstandsheizelement vollständig vom Netz, d.h., von der zum Betrieb des Fluidwärmers eingesetzten Wechselspannungsquelle, getrennt werden. Eine Stromleitung über den Funktionserdungsleiter wird dabei aus Sicherheitsgründen zusätzlich durch den in den geöffneten Schaltzustand überführten Schalter der Funktionserdungsleitung verhindert.

Nach der Erfindung können für einen Betriebszustand des Fluidwärmers mit aktiviertem Widerstandsheizelement und für einen Betriebszustand mit deaktiviertem Widerstandsheizelement unterschiedliche minimale Schwellenstromstärken $I_{TOn}$, $I_{TOff}$ für den Ableitstrom vorgegeben sein. Der erfindungsgemäße Fluidwärmer bietet dadurch überdies auch bei Betrieb mit anderen elektrischen medizinischen Instrumenten/Geräten Sicherheitsvorteile. So kann ein Abfallen des über den Funktionserdungsleiter abgeleiteten Ableitstroms auf/unter den minimalen Schwellenstrom auf eine schwerwiegende Funktionsstörung des anderen Instruments/Geräts hinweisen, bei der Ableitströme überwiegend oder auch insgesamt über das jeweils andere Instrument/Gerät abgeführt werden. Durch das Trennen einer elektrisch leitenden Verbindung zwischen dem Widerstandsheizelement und der Wechselspannungsquelle sowie zwischen dem Wärmeübertrager und dem funktionellen Erdungsanschluss können auf diese Weise ggf. elektrisch fließende und sich möglicherweise letal auswirkende Fehlerströme über den Fluidwärmer vermieden werden. Die minimalen Schwellenstromstärken sind bevorzugt kleiner als die vorstehend erläuterten maximalen Schwellenstromstärken.

Das Zeitintervall, innerhalb dessen die Schalter in ihren geöffneten Schaltzustand überführbar sind, ist nach der Erfindung bevorzugt auf die Periodendauer der von der eingesetzten Wechselspannungsquelle bereitgestellten Wechselspannung ausgerichtet. Das Zeitintervall ist dabei in jedem Fall kürzer als eine Periodendauer T, bevorzugt kürzer als eine Halbperiodendauer $T_{halb}$, der von der Wechselspannungsquelle bereitgestellten Wechselspannung (=Netzspannung). Die maximale Schaltzeit der Schalter beträgt, beispielsweise bei einer Wechselspannung mit 50 Hz, vorzugsweise weniger als 20 ms, bevorzugt gleich oder weniger als 16 ms, ganz besonders bevorzugt weniger als 10 ms.

Die ansteuerbaren Schalter können nach der Erfindung insbesondere als elektronische Schalter, beispielsweise als TRIAC (Zweirichtungs-Thyristortriode) oder MOSFET (Metall-Oxid-Halbleiter-Feldeffekttransistor), ausgebildet sein. Im Falle der elektronischen Schalter können besonders kurze Schaltzeiten realisiert werden. Aufgrund der fehlenden Schaltkontakte können dadurch zudem ein

Verschleiß der Schalter sowie unerwünschte Prelleffekte vermieden werden. Die Schalter können nach einer Weiterbildung der Erfindung

auch jeweils als Relais ausgebildet sein. Unter sicherheitstechnischen Aspekten sind die Schalter vorzugsweise jeweils doppelt, d.h., durch jeweils zwei in Serie angeordnete Einzelschalter, ausgeführt, die synchron betätigbar sind.

Die Steuereinrichtung kann insbesondere einen optischen und/oder akustischen Signalgeber aufweisen. Dadurch kann einerseits der geöffnete Schaltzustand der Schalter, d.h., der aktive Schutzmodus des Fluidwärmers, angezeigt werden. Funktionsstörungen am Fluidwärmer oder auch an anderen elektrischen Geräten können dadurch umgehend erkannt und von einer Bedienperson des Fluidwärmers beseitigt werden. Zur erleichterten Differenzierung der Auslöseursache, und/oder Anzeige des Normalen Betriebsmodus (Temperiermodus) des Fluidwärmers können auch mehrere optische/akustische Signalgeber vorgesehen sein, die sich vorzugsweise in ihrem ausgegebenen Signal voneinander, beispielsweise farblich, unterscheiden.

Nach einem Einschalten des Fluidwärmers und vor einem Start des Temperiervorgangs ist derjenige Netzanschlussleiter des Widerstandsheizelements, der außenleiterseitig an die zum Betrieb des Fluidwärmers vorgesehene Wechselspannungsquelle stromführend angeschlossen ist, d.h., unter Wechsel- bzw. Netzspannung steht, vorzugsweise durch die Steuereinrichtung bestimmbar. Dies kann nach der Erfindung insbesondere im Wege einer entsprechend programmierten oder einer hierzu hardwaremäßig entsprechend ausgestalteten Steuereinrichtung realisiert sein. Der stromführende Schalter des außenleiterseitig angeschlossenen Netzanschlussleiters ist vorzugsweise durch die Steuereinrichtung für das Aktivieren/Deaktivieren des Widerstandsheizelements während des Temperiervorgangs alleinig ansteuerbar.

Der Wärmeübertrager kann insbesondere eine Aufnahme, beispielsweise einen Einschubkanal, für ein Behältnis, wie beispielsweise einen Fluidbeutel oder eine Fluidkassette, der Fluidleitung aufweisen. Dadurch wird ein großflächiger Kontakt des Wärmeübertragers mit dem Behältnis und somit eine optimale Wärmeübertragung auf das im Behältnis strömende Fluid ermöglicht.

Es versteht sich, dass der Fluidwärmer auch zwei, drei oder mehr Widerstandsheizelemente aufweisen kann. Die Widerstandsheizelemente können dabei zum Temperieren der Fluidleitung/des medizinischen Fluid gemeinsam oder auch unabhängig voneinander aktivierbar/deaktivierbar sein.

Im Hinblick auf ein effizientes Temperieren des medizinischen Fluids weist der Fluidwärmer vorzugsweise eine Abgabeleistung von zumindest 0,15 Kilowatt, bevorzugt von mehr als 0,25 Kilowatt auf.

Nachfolgend wird die Erfindung anhand eines in der Zeichnung wiedergegebenen Ausführungsbeispiels näher erläutert.

Die gezeigte und beschriebene Ausführungsform ist nicht als abschließende Aufzählung zu verstehen, sondern hat vielmehr beispielhaften Charakter für die Schilderung der Erfindung.

In der Zeichnung zeigen:

Fig. 1 ein Blockschaltbild eines erfindungsgemäßen Fluidwärmers mit einem Widerstandsheizelement für Wechselspannung, das mit einem funktionsgeerdeten Wärmeübertrager wärmeleitend gekoppelt ist;

Fig. 2 den Fluidwärmer aus Fig. 1 im gemeinsamen Betrieb mit einem Dialysegerät;

Fig. 3 ein Blockschaltbild eines erfindungsgemäßen Fluidwärmers, der eine elektrische Kompensationseinrichtung zum Kompensieren eines durch das Widerstandsheizelement im Wärmeübertrager induzierten Ableitstroms aufweist;

Fig. 4 für die Regelung der Fluidwärmer gemäß Fign. 1 bis 3 vorgegebene Schwellenstromstärken für einen messtechnisch zu erfassenden Ableitstrom über den Funktionserdungsleiter des Wärmeübertragers bei aktiviertem Widerstandsheizelement;

Fig. 5 für die Regelung der Fluidwärmer gemäß Fign. 1 bis 3 vorgegebene Schwellenstromstärken für einen messtechnisch zu erfassenden Ableitstrom über den Funktionserdungsleiter bei deaktiviertem Widerstandsheizelement; und

Fig. 6 ein Blockschaltbild mit einzelnen Schritten des erfindungsgemäßen Verfahrens zum Betreiben der Fluidwärmer aus Fign. 1 bis 3.

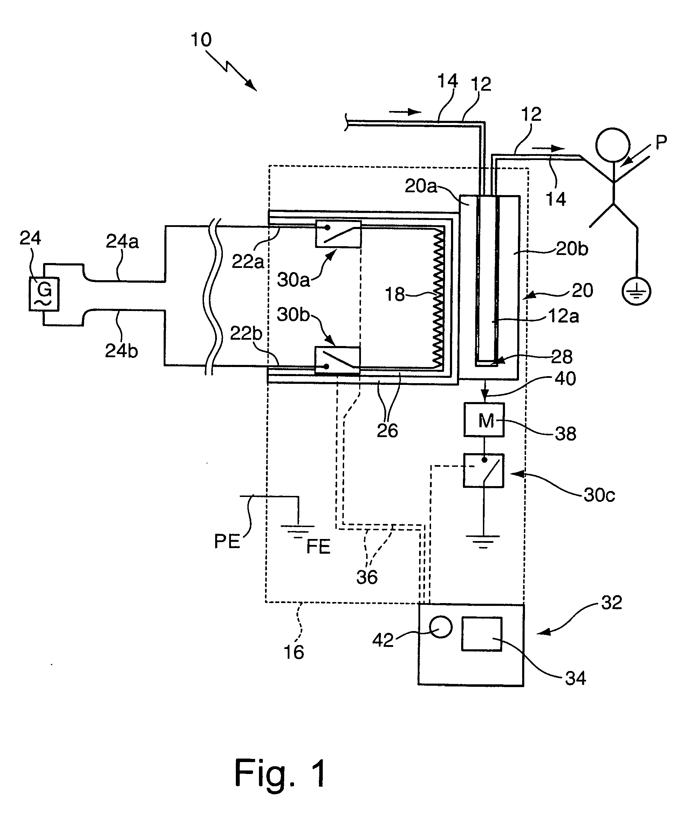

**Fig. 1** zeigt das Blockschaltbild eines Fluidwärmers **10** zum Temperieren eines in einer Fluidleitung **12** geführten medizinischen Fluids **14.** Der Fluidwärmer 10 weist ein Gehäuse **16** mit zwei oder mehr in dem Gehäuse **16** angeordneten Widerstandsheizelementen **18** auf, die mit einem Wärmeübertrager **20** thermisch gekoppelt, d.h., mit diesem wärmeleitend verbunden, sind. In der Fig. 1 ist nur eines der beiden Widerstandsheizelemente 18 wiedergegeben. Die Widerstandsheizelemente 18 sind vorliegend jeweils als Heizspirale ausgebildet, können aber auch eine andere Formgebung aufweisen.

Jedes Widerstandsheizelement 18 weist einen ersten und einen zweiten Netzanschlussleiter **22a, 22b** auf, die einem elektrischen Anschluss des Widerstandsheizelements 18 an eine Netzspannung, d.h, an eine externe Wechselspannungsquelle **24,** dienen.

Die für den Betrieb des Widerstandsheizelements 18 vorgesehene Wechselspannungsquelle 24 weist eine übliche länderspezifische Betriebsspannung von beispielsweise 230 V $\pm$ 10%, und eine Netzfrequenz von ungefähr 50Hz auf.

Der in der Fig. obere Netzanschlussleiter 22a des gezeigten Widerstandsheizelements 18 ist vorliegend mit einem Außenleiter **24a** der Wechselspannungsquelle 24 und der zweite Netzanschlussleiter 22b mit einem Neutralleiter **24b** der Wechselspannungsquelle 24 elektrisch leitend verbunden.

Die beiden Netzanschlussleiter 22a, 22b und das Widerstandsheizelement 18 bilden einen Netzstromkreis des Fluidwärmers 10, der mittels einer verstärkten, hier doppelten, Isolierung **26** gegenüber dem Gehäuse 16 elektrisch isoliert ist.

Der Wärmeübertrager 20 ist mittels eines Funktionserdungsleiters **FE** funktionsgeerdet und als ein im Betrieb berührbares Bauteil ausgelegt. Der Funktionserdungsleiter FE ist mit dem Schutzleiter **PE** verbunden. Der Wärmeübertrager 20 weist zwei zueinander parallel ausgerichtete Wärmeübertragerplatten **20a, 20b** auf, die unter Ausbildung einer Aufnahmekammer **28** voneinander beabstandet angeordnet sind. In der Aufnahmekammer 28 ist ein beutelförmig erweitertes Behältnis **12a,** das fluidtechnisch mit der Fluidleitung 12 verbunden ist, eingeschoben bzw. eingelegt. Das beutelförmige Behältnis 12a kann in bekannter Weise integraler Bestandteil der Fluidleitung sein und liegt an den beiden Wärmeübertragerplatten 20a, 20b des Wärmeübertragers 20 im wesentlichen vollflächig an. Dadurch wird eine optimale Wärmeübertragung auf das im Behältnis 12a strömend geführte Fluid 14 gewährleistet.

Die Fluidleitung 12 ist als eine im medizinischen Sektor an sich bekannte Infusionsleitung ausgebildet und besteht aus Kunststoff. Die Fluidleitung ist einenends (über einen nicht näher wiedergegebenen venösen Zugang /Venenverweilkatheter) an einen Blutkreislauf (nicht gezeigt) einer zu behandelnden Person (Patienten) **P,** angeschlossen. Das Fluid 14 steht somit vorliegend mit dem Blutkreislauf der Person P unter Umgehung des in der Regel hochohmigen Hautwiderstands in einem elektrisch leitfähigen Kontakt.

Wie in Fig. 1 gezeigt ist, sind die beiden Netzanschlussleiter 22a, 22b und der Funktionserdungsleiter FE jeweils mit einem elektrisch ansteuerbaren Schalter **30a, 30b, 30c** versehen. Die Schalter 30a, 30b, 30c sind jeweils zwischen einem eingeschalteten (geschlossenen) und einem in der Figur gezeigten ausgeschalteten (geöffneten) Schaltzustand umschaltbar. Im eingeschalteten Schaltzustand sind die Schalter 30a, 30b, 30c stromleitend, d.h., deren Schaltereingang ist mit dem Schalterausgang niederohmig elektrisch verbunden. Im ausgeschalteten Schaltzustand sind die Schalter 30a, 30b, 30c nicht stromleitend, d.h., deren Ein- und Ausgang sind im ausgeschalteten Schaltzustand jeweils voneinander elektrisch isoliert. Die Schalter 30a, 30b, 30c sind vorliegend jeweils als Relais ausgebildet, können aber auch als MOSFET (Metall-Oxid-Halbleiter-Feldeffekttransistor), als TRIAC (Zweirichtungs-Thyristortriode) oder dergl., ausgebildet sein.

Eine Steuereinrichtung 32 mit einem Anzeige- und Bedienfeld **34** dient vorliegend einer Steuerung aller Betriebsparameter des Fluidwärmers 10. Die Steuereinrichtung 32 ist mit den Schaltern 30a, 30b, 30c über Steuerleitungen **36** verbunden. Die Steuereinrichtung 32 weist einen Sensor **38** zum messtechnischen Erfassen eines über den Funktionserdungsleiter FE abgeleiteten Ableitstroms **40** auf.

Das vorgegebene Zeitintervall, innerhalb dessen die Schalter 30a, 30b, 30c durch die Steuereinrichtung 32 in ihren geöffneten Schaltzustand überführbar sind, ist immer kleiner als eine Periodendauer, vorzugsweise kleiner als eine Halbperiodendauer, der zum Betrieb der Widerstandsheizelemente 18 eingesetzten Wechselspannung. Das Zeitintervall ist bei einer Netzfrequenz von 50 Hz insbesondere kleiner oder gleich 16ms.

Die Steuereinrichtung 32 weist einen Signalgeber **42** zur Anzeige eines optischen und/oder akustischen Alarmsignals auf.

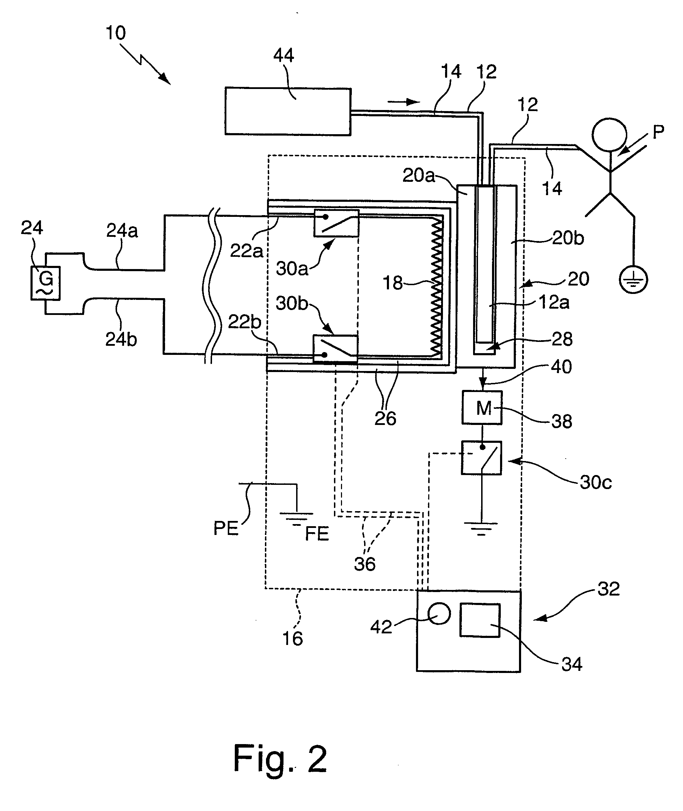

In **Fig. 2** ist der Fluidwärmer 10 bei einem kombinierten Einsatz mit einem weiteren medizinischen Gerät **44,** hier einem Dialysegerät, an einer zu behandelnden Person P gezeigt. Dialysegeräte werden bekanntlich bei einer Nierenersatzbehandlung zum Entfernung gelöster Substanzen sowie bedarfsweise eines definierten Wasseranteils aus dem Blut am Patienten eingesetzt. Das dialysierte Blut strömt vom Dialysegerät über den Fluidwärmer 10, in dem das Blut erwärmt wird, zu der Person P.

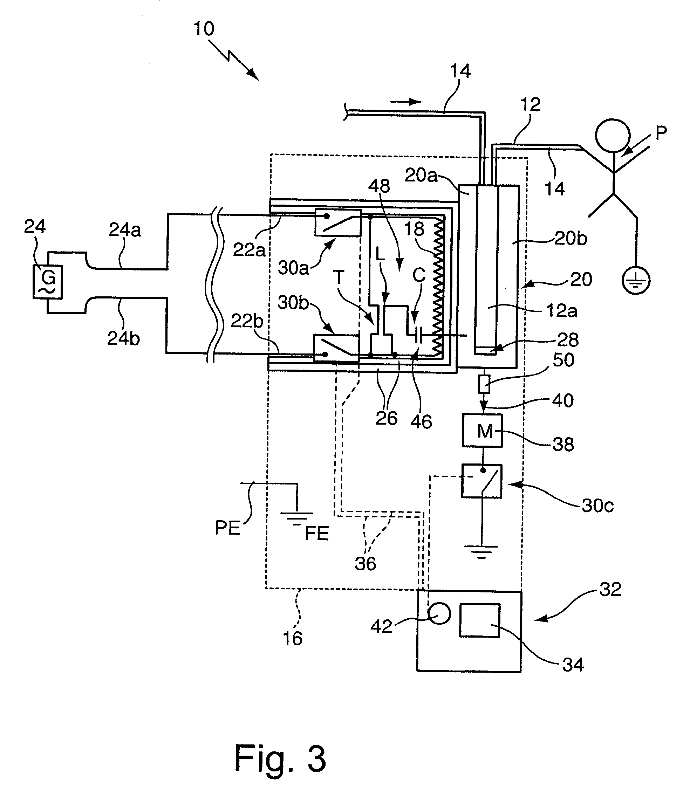

Fig. 3 zeigt einen Fluidwärmer 10, der gegenüber dem vorstehend erläuterten Fluidwärmer zusätzlich eine elektrische Kompensationseinrichtung **46** für den durch den Betrieb der Widerstandsheizelemente 18 induzierten Ableitstrom 40 aufweist. Die Kompensationseinrichtung 46 weist einen LC-Schwingkreis **48** mit einer Spule L und einem Kondensator C auf. Der LC-Schwingkreis 48 ist an den Wärmeübertrager 20 und den Netzanschlussleiter 22b angeschlossen, der neutralleiterseitig an die Wechselspannungsquelle 24 angeschlossenen ist. Die Spule L und der Kondensator C des LC-Schwingkreises 48 sind vorliegend in Reihe geschaltet. Die Spule L ist durch eine Sekundärseite eines Trafos T gebildet, der primärseitig an die beiden Netzanschlussleiter 22a, 22b angeschlossen ist. Beim gemeinsamen Betrieb der Widerstandsheizelemente 20 und des Transformators T an der Netzspannung der Wechselspannungsquelle 24 wird der LC-Schwingkreis 48 zu einer zwangsläufigen elektrischen Schwingung angeregt. Aufgrund einer Phasenverschiebung des Stroms im LC-Schwingkreis 48 gegenüber dem Heizstrom der Widerstandsheizelemente 20 wird der durch die Widerstandsheizelemente induzierte Ableitstrom 40 zumindest teilweise kompensiert. Der oben beschriebene Schwingkreis 48 kann auch mit elektronischen Bauelementen aufgebaut sein (z.B. unter Verwendung eines Mikroprozessors). Dem Sensor 38 ist bei diesem Ausführungsbeispiel des Fluidwärmers 10 ein Widerstandselement 50 vorgeschaltet sein.

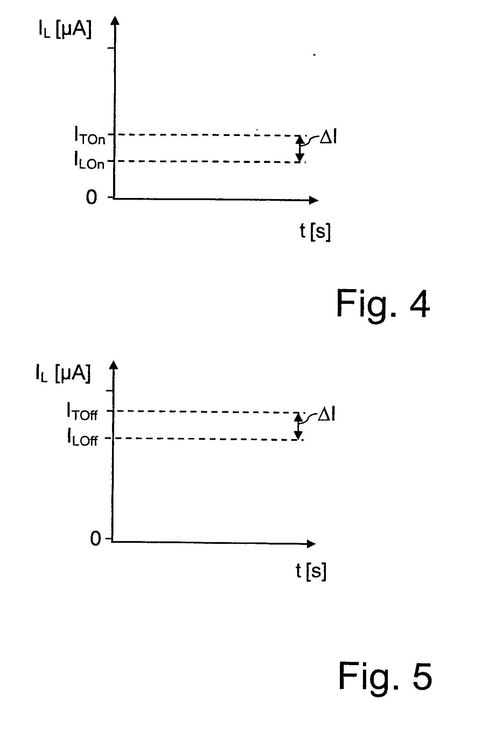

In **Fig. 4** ist eine maximale Schwellenstromstärke $I_{TOn}$ und eine minimale Schwellenstromstärke $I_{LOn}$ für die Stromstärke $I_L$ des Ableitstroms 40 verdeutlicht, anhand derer der jeweilige Schaltzustand der Schalter 30a, 30b; 30c durch die Steuereinrichtung 32 bei aktiviertem Widerstandsheizelement 18 (Fign. 1 und 2) geregelt wird. Die Differenz zwischen dem maximalen Schwellenstromstärke $I_{Ton}$ und $I_{LON}$ entspricht einer Differenzstromstärke $\Delta I$.

In **Fig. 5** ist eine maximale Schwellenstromstärke $I_{TOff}$ und eine minimale Schwellenstromstärke $I_{LOff}$ für die Stromstärke $I_L$ des über den Funktionserdungsleiter FE abgeleiteten Ableitstroms 40 verdeutlicht, anhand derer der jeweilige Schaltzustand der Schalter 30a, 30b; 30c durch die Steuereinrichtung 32 beim Temperiervorgang bei deaktiviertem Widerstandsheizelement 18 (Fign. 1 und 2) geregelt wird. Die Differenz zwischen der maximalen Schwellenstromstärke $I_{TOff}$ und der minimalen Schwellenstromstärke $I_{LOff}$ entspricht der Differenzstromstärke $\Delta I$. Die Differenzstromstärke $\Delta I$ kann benutzerseitig vorgegeben sein.

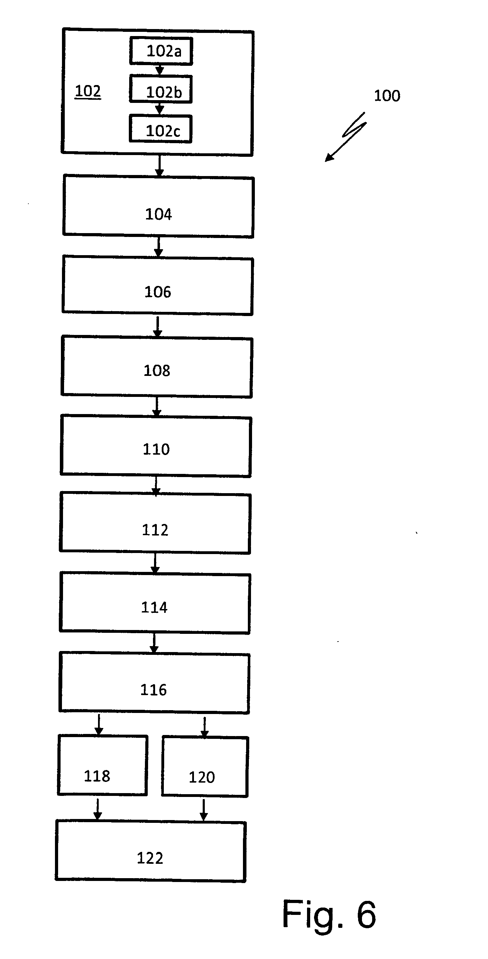

Nachfolgend wird das erfindungsgemäße Verfahren **100** zum Betrieb des Fluidwärmers 10 aus Fig. 1 und 2 unter zusätzlicher Bezugnahme auf **Fig. 6** erläutert.

Die Steuereinrichtung 32 (Fig. 1, 2) des Fluidwärmers 10 ist vorliegend zur Ausführung der einzelnen Verfahrensschritte programmiert. Die Steuereinrichtung kann jedoch auch durch ihre rein hardwaremäßige Ausgestaltung die Durchführung des Verfahrens 100 ermöglichen.

Nach einem Anschließen des Fluidwärmers 10 an die Wechselspannungsquelle 24 und einem Einschalten des Fluidwärmers 10 befindet sich die Steuereinrichtung 32 zunächst in einem Standby Modus.

Vor der Inbetriebnahme der Heizung identifiziert die Steuereinrichtung 32 in einem ersten Schritt **102** denjenigen Netzanschlussleiter 22a, 22b, der außenleiterseitig an die Wechselspannungsquelle 24 angeschlossen ist und daher bei geschlossenem zugehörigen Schalter 30a, 30b, 30c unter Spannung steht.

Dazu wird in einem ersten Teilschritt **102a** mittels des Sensors 38 die Stromstärke $I_{L1}$ des Ableitstroms 40 über den Funktionserdungsleiter FE bei geöffnetem Schalter 30a des (hier außenleiterseitig an die Wechselspannungsquelle 24 angeschlossen) ersten Netzanschlussleiters 22a und geschlossenem Schalter 30b des (vorliegend neutralleiterseitig an die Wechselspannungsquelle 24 angeschlossenen) zweiten Netzanschlussleiters 22b bestimmt und von der Steuereinrichtung 32 abgespeichert.

In einem zweiten Teilschritt **102b** wird mittels des Sensors 38 eine Stromstärke $I_{L_2}$ des Ableitstroms 40 über den Funktionserdungsleiter FE bei geschlossenem Schalter 30a des ersten Netzanschlussleiters 22a und bei geöffnetem Schalter 30b des zweiten Netzanschlussleiters 22b bestimmt und von der Steuereinrichtung 32 abgespeichert. Es versteht sich, dass der Schalter 30c des Funktionserdungsleiters FE bei den beiden vorgenannten Teilschritten 102a, 102b jeweils geschlossen ist.

In einem dritten Teilschritt **102c** wird der außenleiterseitig angeschlossene Netzanschlussleiter 22a, 22b anhand der erfassten und abgespeicherten Stromstärken $I_{L_1}$, $I_{L_2}$ identifiziert. Der außenleiterseitig angeschlossene Netzanschlussleiter 22a, 22b ist derjenige Netzanschlussleiter 22a, 22b, bei dem der Ableitstrom 40 im stromführenden (geschlossenen) Schaltzustand des dem jeweiligen Netzanschlussleiter 22a, 22b zugeordneten Schalters 30a, 30b der jeweilig größere ist.

Der Schalter 30a des Neutralleiters 22a wird von der Steuereinrichtung 32 in einem weiteren Schritt **104** als anzusteuernder Schalter 30a, 30b, 30c für ein Aktivieren/Deaktivieren des Widerstandsheizelements 18 während des Temperiervorgangs des Fluids 14 vorgegeben.

Die Steuereinrichtung 32 bestimmt in einem weiteren Schritt **106** eine Stromstärke $I_{LOn}$ über den Funktionserdungsleiter FE bei einer maximalen Heizleistung (Vollast) des Widerstandsheizelements 18.

Die Steuereinrichtung 32 definiert in einem nachfolgenden Schritt **108** die maximale Schwellenstromstärke $I_{TOn}$ als Summe der Stromstärke $I_{LOn}$ bei aktiviertem Widerstandsheizelement und der Differenzstromstärke $\Delta I$:

$$I_{TOn} = I_{LOn} + \Delta I \qquad\qquad (I)$$

Die Steuereinrichtung 32 gibt in einem weiteren Schritt **110** die in dieser Weise definierte maximale Schwellenstromstärke $I_{TOn}$ für den Temperierbetrieb des Fluidwärmers 10 bei aktivierten Widerstandsheizelementen 18 vor. Man beachte, dass sich die beiden Schalter 30a, 30b der Netzanschlussleiter 22a, 22b bei aktiviertem Widerstandsheizelement 18 jeweils in ihrem geschlossenen Schaltzustand befinden, so dass das Widerstandsheizelement 18 von einem von der Wechselspannungsquelle 24 bereitgestellten Heizstrom durchflossen ist.

Die Steuereinrichtung 32 definiert in einem weiteren Schritt **112** die maximale Schwellenstromstärke $I_{TOff}$ für den über den Funktionserdungsleiter FE abgeführten Ableitstrom 40 bei einem Betriebszustand des Fluidwärmers 10 mit deaktivierten Heizelementen 18.

Wie vorstehend erläutert, befindet sich der Schalter 30a des mit dem Neutralleiter 24a der Wechselspannungsquelle 24 verbundenen ersten Netzanschlussleiters 22a bei deaktiviertem Widerstandsheizelement 18 in seinem geöffneten Schaltzustand und der Schalter 30b des außenleiterseitig angeschlossenen Netzanschlussleiters 22b in seinem geschlossenen Schaltzustand.

Die Steuereinrichtung definiert die maximale Schwellenstromstärke $I_{TOff}$ als Summe der Stromstärke $I_{LOff}$ und der Differenzstromstärke $\Delta I$:

$$I_{TOff} = I_{LOff} + \Delta I \qquad\qquad (II)$$

Die maximale Schwellenstromstärke $I_{TOff}$ wird von der Steuereinrichtung 32 in einem weiteren Schritt **114** für den Ableitstrom 40 und den Temperierbetrieb des Fluidwärmers 10 mit deaktiviertem Heizelement 18 vorgegeben.

Die Differenzstromstärke $\Delta I$ kann grundsätzlich zwischen 1 Mikroampere und maximal 50 Mikroampere betragen und kann in der Steuereinrichtung 32 als Fixwert hinterlegt (abgespeichert) sein. Die Differenzstromstärke $\Delta I$ kann auch von einer Bedienperson, beispielsweise über das Bedienfeld 34 der Steuereinrichtung, verändert bzw. manuell ein- und vorgegeben werden.

Es versteht sich, dass bei einem zeitgleichen Einsatz des Fluidwärmers 10 am Patienten P mit einem oder mehreren anderen Geräten (Fig. 2) eine kleinere Differenzstromstärke $\Delta I$ gewählt wird, als bei dem in Fig. 1 wiedergegebenen stand-alone Betrieb des Fluidwärmers 10.

Die Steuereinrichtung 32 kann den Ableitstrom 40 während des Temperiervorgangs in einem weiteren Schritt **116** über den Funktionserdungsleiter FE, vorzugsweise kontinuierlich, überwachen.

Die Steuereinrichtung 32 vergleicht dabei den Ableitstrom 40 bei aktiviertem Widerstandsheizelement 18 mit der maximalen Schwellenstromstärke $I_{TOn}$ und bei deaktiviertem Widerstandsheizelement mit der maximalen Schwellenstromstärke $I_{TOff}$. Sobald der Ableitstrom 40 die für den jeweiligen Betriebszustand des Fluidwärmers (aktiviertes/deaktiviertes Widerstandsheizelement 18) vorgegebene maximale Schwellenstromstärke $I_{TOn}$, $I_{TOff}$ erreicht oder übersteigt, überführt die Steuereinrichtung 32 die Schalter 30a, 30b des ersten und des zweiten Netzanschlussleiters 22a, 22b sowie den Schalter 30c des Funktionserdungsleiters FE in einem weiteren Schritt **118** innerhalb des vorgegebenen Zeitintervalls gemeinsam in ihren geöffneten Schaltzustand, so dass sich der Fluidwärmer 10 in einem aktivierten Schutzmodus befindet.

Die Steuereinrichtung 32 kann darüber hinaus die Schalter 30a, 30b, 30c im Temperierbetrieb des Fluidwärmers 10 bei einem Ableitstrom 40, der kleiner oder gleich der vorgegebenen minimalen Schwellenstromstärke $I_{LOn}$, $I_{LOff}$ ist, in einem weiteren Schritt **120** innerhalb des vorgegebenen Zeitintervalls gemeinsam in ihren geöffneten Schaltzustand überführen. Im stand-alone Betrieb des Fluidwärmers 10 kann so eine fehlerhafte Funktionserdung FE des Wärmeübertragers 20 festgestellt und eine Gefährdung des Patienten P durch unerwünscht hohe Patientenableitströme entgegengewirkt werden. Bei einem zeitgleichen Einsatz des Fluidwärmers 10 mit einem weiteren Gerät am Patienten P können darüber hinaus Störungen des anderen Geräts, bei dem kapazitive Ableitströme überwiegend oder alleinig über das andere Gerät abgeführt werden, erkannt werden. Der Fluidwärmer 10 befindet sich auch hier in seinem aktivierten Schutzmodus.

Der aktivierte Schutzmodus des Fluidwärmers 10 wird in einem abschließenden Schritt **122** durch den Signalgeber 42 angezeigt.

Die Erfindung betrifft einen Fluidwärmer 10 zum Temperieren eines in einer Fluidleitung 12 geführten medizinischen Fluids 14. Der Fluidwärmer 10 umfasst

- ein Widerstandsheizelement 18 für Wechselspannung, das einen ersten und einen zweiten elektrischen Netzanschlussleiter 22a, 22b aufweist;
- einen Wärmeübertrager 20, der mit dem Widerstandsheizelement 18 thermisch leitend verbunden ist;
- einen Funktionserdungsleiter FE zum Funktionserden des Wärmeübertragers 20;
- einen Sensor 38 zum Bestimmen einer jeweiligen Stromstärke eines Ableitstroms 40 über den Funktionserdungsleiter FE; und
- eine Steuereinrichtung, wobei

die beiden Netzanschlussleiter 22a, 22b und der Funktionserdungsleiter FE jeweils mit Schaltern 30a, 30b, 30c versehen sind, und wobei die Schalter 30a, 30b, 30c bei einem Ableitstrom 40, der größer oder gleich einer definierten maximalen Schwellenstromstärke $I_{TOn}$, $I_{TOff}$ ist, durch die Steuereinrichtung innerhalb eines vorgegebenen Zeitintervalls gemeinsam in ihren geöffneten Schaltzustand überführbar sind.

## Patentansprüche

**1.** Fluidwärmer (10) zum Temperieren eines in einer Fluidleitung (12) geführten medizinischen Fluids (14), mit

- einem Widerstandsheizelement (18) für Wechselspannung, das einen ersten und einen zweiten elektrischen Netzanschlussleiter (22a, 22b) aufweist; mit
- einem Wärmeübertrager (20), der mit dem Widerstandsheizelement (18) thermisch gekoppelt ist,
- einem Funktionserdungsleiter (FE), der mit dem Wärmeübertrager (20) elektrisch leitend verbunden ist, mit
- einem Sensor (38) zum Bestimmen einer jeweiligen Stromstärke ($I_L$) eines Ableitstroms (40) über den Funktionserdungsleiter (FE),
- mit einer elektrischen oder einer elektronischen
Kompensationseinrichtung (46) zur Kompensation des durch das Widerstandsheizelement (18) induzierten Ableitstroms (40) und mit
- einer Steuereinrichtung (32), wobei
die beiden Netzanschlussleiter (22a; 22b) und der Funktionserdungsleiter (FE) jeweils mit Schaltern (30a; 30b; 30c) versehen sind, und wobei
die die Schalter (30a; 30b; 30c) bei einem Ableitstrom (40), der größer oder gleich einer definierten maximalen Schwellenstromstärke ($I_{Ton}$, $I_{TOff}$) ist, mittels der Steuereinrichtung (32) innerhalb eines vorgegebenen Zeitintervalls gemeinsam in ihren geöffneten Schaltzustand überführbar sind.

**2.** Fluidwärmer nach Anspruch 1, **dadurch gekennzeichnet, dass** für einen Betriebszustand des Fluidwärmers (10) mit aktiviertem Widerstandsheizelement (18) und für einen Betriebszustand mit deaktiviertem Widerstandsheizelement (18) unterschiedliche Schwellenstromstärken ($I_{TOn}$, $I_{TOff}$) vorgegeben sind.

3. Fluidwärmer nach einem der vorhergehenden Ansprüche, **dadurch gekennzeichnet, dass** die maximale Schwellenstromstärke ($I_{TOn}$) der Summe einer Stromstärke ($I_{LOn}$) des Ableitstroms (40) bei einer maximalen Heizleistung des Widerstandsheizelements (18) und einer, vorzugsweise frei vorgebbaren, Differenzstromstärke ($\Delta I$) entspricht.

4. Fluidwärmer nach einem der vorhergehenden Ansprüche, **dadurch gekennzeichnet, dass** die maximale Schwellenstromstärke ($I_{TOff}$) der Differenzstromstärke ($\Delta I$) oder der Summe der Differenzstromstärke ($\Delta I$) und einer Stromstärke ($I_{LOff}$) des Ableitstroms (40) bei geschlossenem Schalter (30a; 30b; 30c) des außenleiterseitig an eine Wechselspannungsquelle (24) angeschlossenen Netzanschlussleiters (22a, 22b) und geöffnetem Schalter (30a; 30b; 30c) des neutralleiterseitig an die Wechselspannungsquelle (24) angeschlossenen Netzanschlussleiters (22a;22b) des Widerstandsheizelements (18) entspricht.

5. Fluidwärmer nach einem der Ansprüche 3 oder 4, **dadurch gekennzeichnet, dass** die Differenzstromstärke ($\Delta I$) zwischen 1 Mikroampere und maximal 50 Mikroampere beträgt.

6. Fluidwärmer nach einem der vorhergehenden Ansprüche, **dadurch gekennzeichnet**, dass-die Schalter (30a; 30b; 30c) bei einem Ableitstrom (40), der kleiner oder gleich einer vorgegebenen minimalen Schwellenstromstärke ($I_{LOn}$, $I_{LOff}$) ist, innerhalb des vorgegebenen Zeitintervalls gemeinsam in ihren geöffneten Schaltzustand überführbar sind.

7. Fluidwärmer nach Anspruch 6, **dadurch gekennzeichnet, dass** für einen Betriebszustand des Fluidwärmers (10) mit aktiviertem Widerstandsheizelement (18) und für einen Betriebszustand mit deaktiviertem Widerstandsheizelement (18) unterschiedliche Schwellenstromstärken ($I_{TOn}$, $I_{TOff}$) vorgegeben sind.

8. Fluidwärmer nach einem der vorhergehenden Ansprüche, **dadurch gekennzeichnet, dass** das vorgegebene Zeitintervall, innerhalb dessen die Schalter (30a; 30b; 30c) durch die Steuereinrichtung (32) in den geöffneten Schaltzustand überführbar sind, kleiner als eine Periodendauer, vorzugsweise kleiner als eine Halbperiodendauer, der zum Betrieb des Widerstandsheizelements (18) eingesetzten Wechselspannung ist.

9. Fluidwärmer nach einem der vorhergehenden Ansprüche, **dadurch gekennzeichnet, dass** die Schalter (30a; 30b; 30c) als eine Zweirichtungs-Thyristortriode (TRIAC), als ein Metall-Oxid-Halbleiter-Feldeffekttransistor (MOSFET) oder als ein Relais ausgebildet sind und/oder dass die Schalter jeweils doppelt ausgeführt sind..

10. Fluidwärmer nach einem der vorhergehenden Ansprüche, **dadurch gekennzeichnet, dass** derjenige Netzanschlussleiter (22a; 22b), der außenleiterseitig stromführend an eine Wechselspannungsquelle (24) angeschlossen ist, durch die die Steuereinrichtung (32) ist, identifizierbar ist und dass der Schalter (30a; 30b; 30c) dieses Netzanschlussleiters (22a; 22b) während des Betriebs des Fluidwärmers (10) von der Steuereinrichtung (32) zum Aktivieren/Deaktivieren des Widerstandsheizelements (18) ansteuerbar ist

11. Fluidwärmer nach Anspruch 1, **dadurch gekennzeichnet, dass** die Kompensationseinrichtung (46) einen LC-Schwingkreis (48) mit einer Spule (L) und einem Kondensator (C) umfasst.

12. Fluidwärmer nach Anspruch 11, **dadurch gekennzeichnet, dass** die Spule (L) des LC-Schwingkreises (48) als Sekundärseite eines Transformators (T) ausgebildet ist, der primärseitig an die Netzanschlussleiter (22a, 22b) des Widerstandsheizelements (18) angeschlossen ist.

13. Fluidwärmer nach einem der vorhergehenden Ansprüche, **dadurch gekennzeichnet, dass** der Wärmeübertrager (20) eine Aufnahmekammer (28) für ein beutelförmiges Behältnis (12a) der Fluidleitung (12) aufweist.

14. Fluidwärmer nach einem der vorhergehenden Ansprüche, **gekennzeichnet durch** zwei oder mehr Widerstandsheizelemente (18).

15. Fluidwärmer nach einem der vorhergehenden Ansprüche, **gekennzeichnet durch** eine Abgabeleistung von insgesamt zumindest 0,15 KW, bevorzugt von mehr als 0,2KW.

16. Verfahren (100) zum Betrieb eines Fluidwärmers (10) zum Temperieren eines in einer Fluidleitung (12) strömenden Fluids (14), umfassend:

   - ein Widerstandsheizelement (18), mit einem ersten und einem zweiten elektrischen Netzanschlussleiter (22a;

22b) zum Anschließen des Widerstandsheizelements (18) an eine Wechselspannungsquelle (24),
- einen Wärmeübertrager (20), der mit dem Widerstandsheizelement (18) thermisch gekoppelt ist, zum Übertragen von Wärme auf das in einem Behältnis (12a) der Fluidleitung (12) strömende Fluid (14),
- einen mit dem Wärmeübertrager (20) verbundenen Funktionserdungsleiter (FE) zur funktionellen Erdung des Wärmeübertragers (20);
- einen Sensor (38) zum Erfassen eines jeweiligen Ableitstroms 40 über den Funktionserdungsleiter (FE),
- eine elektrische oder eine elektronische Kompensationseinrichtung (46) zur Kompensation des durch das Widerstandsheizelement (18) induzierten Ableitstroms (40) und
- eine Steuereinrichtung (32) zum Ansteuern von Schaltern (30a; 30b; 30c), mit denen die Netzanschlussleiter (22a; 22b) und der Funktionserdungsleiter (FE) jeweils verbunden sind,
aufweisend die folgenden Schritte:
- Vorgeben einer maximalen Schwellenstromstärke ($I_{TOn}$, $I_{TOff}$) für einen Ableitstrom (40) über den Funktionserdungsleiter (FE);
- zumindest teilweises Kompensieren des durch das Widerstandsheizelement im Wärmeübertrager induzierten Ableitstroms mittels der Kompensationseinrichtung;
- Vergleichen des mit dem Sensor (38) erfassten Ableitstroms (40) mit der vorgegebenen maximalen Schwellenstromstärke ($I_{TOn}$, $I_{TOff}$); und
- Überführen der Schalter (30a; 30b; 30c) in ihren geöffneten Schaltzustand innerhalb eines vorgegebenen Zeitintervalls bei einem Ableitstrom (40), der gleich oder größer als die vorgegebene maximale Schwellenstromstärke ($I_{TOn}$, $I_{TOff}$) ist.

17. Verfahren nach Anspruch 16, **gekennzeichnet durch** die folgenden weiteren Schritte:

- Vergleichen des mit dem Sensor (38) erfassten Ableitstroms (40) mit einer vorgegebenen minimalen Schwellenstromstärke ($I_{LOn}$, $I_{LOff}$); und
- Überführen der Schalter (30a; 30b; 30c) in ihren geöffneten Schaltzustand innerhalb des vorgegebenen Zeitintervalls bei einem Ableitstrom (40), der kleiner oder gleich der minimalen Schwellenstromstärke ($I_{LOn}$, $I_{LOff}$) ist.

## Claims

1. Fluid warmer (10) for temperature control of a medical fluid (14) guided in a fluid tube (12), with

    - a resistance heating element (18) for alternating voltage, which has a first and a second electrical supply connection conductor (22a, 22b); with
    - a heat transfer element (20) which is thermally coupled to the resistance heating element (18),
    - a functional grounding conductor (FE), which is electrically conductively connected to the heat transfer element (20), with
    - a sensor (38) for determining a present current magnitude ($I_L$) of a leakage current (40) via the functional grounding conductor (FE),
    - with an electric or electronic compensation device (46) for compensation of the leakage current (40) induced by the resistance heating element (18) and with
    - a control device (32), wherein
    the two supply connection conductors (22a; 22b) and the functional grounding conductor (FE) are each equipped with switches (30a; 30b; 30c), and wherein
    the switches (30a; 30b; 30c) can be put into their open switching state jointly within a defined time interval by means of the control device (32) on occurrence of a leakage current (40) that is greater than or equal to a defined maximum threshold current magnitude ($I_{Ton}$, $I_{Toff}$).

2. Fluid warmer according to claim 1, **characterized in that** different threshold current magnitudes ($I_{Ton}$, $I_{Toff}$) are defined for an operating condition of the fluid warmer (10) with an activated resistance heating element (18) and for an operating condition with a deactivated resistance heating element (18).

3. Fluid warmer according to any one of the previous claims, **characterized in that** the maximum threshold current magnitude ($I_{Ton}$) corresponds to the sum of a current magnitude ($I_{Lon}$) of the leakage current (40) for a maximum heating power of the resistance heating element (18) and a preferably freely definable differential current magnitude

($\Delta$I).

4. Fluid warmer according to any one of the previous claims, **characterized in that** the maximum threshold current magnitude ($I_{Toff}$) corresponds to the differential current magnitude ($\Delta$I) or to the sum of the differential current magnitude ($\Delta$I) and a current magnitude ($I_{Loff}$) of the leakage current (40) with a closed switch (30a; 30b; 30c) of the supply connection conductor (22a, 22b) connected to the line conductor of the alternating voltage source (24) and with an open switch (30a; 30b; 30c) of the supply connection conductor (22a; 22b) of the resistance heating element (18) connected to the neutral conductor of the alternating voltage source (24).

5. Fluid warmer according to either one of the claims 3 or 4, **characterized in that** the differential current magnitude ($\Delta$I) is between 1 microampere and no more than 50 microamperes.

6. Fluid warmer according to any one of the previous claims, **characterized in that** the switches (30a; 30b; 30c) can be put into their open switching state jointly within a defined time interval on occurrence of a leakage current (40), which is less than or equal to a defined minimum threshold current magnitude ($I_{Lon}$, $I_{Loff}$).

7. Fluid warmer according to claim 6, **characterized in that** different threshold current magnitudes ($I_{Ton}$, $I_{Toff}$) are defined for an operating condition of the fluid warmer (10) with an activated resistance heating element (18) and for an operating condition with a deactivated resistance heating element (18).

8. Fluid warmer according to any one of the previous claims, **characterized in that** the defined time interval within which the switches (30a; 30b; 30c) can be put into the open switching state by the control device (32) is less than one period, preferably less than one half-period of the alternating voltage used to power the resistance heating element (18).

9. Fluid warmer according to any one of the previous claims, **characterized in that** the switches (30a; 30b; 30c) are constituted as a bidirectional triode thyristor (TRIAC), as a metal-oxide semiconductor field-effect transistor (MOSFET), or as a relay and/or that each of the switches is duplicated.

10. Fluid warmer according to any one of the previous claims, **characterized in that** the supply connection conductor (22a; 22b) that is conductively connected to the line conductor of an alternating voltage source (24) can be identified by the control device (32) and that the switches (30a; 30b; 30c) of this supply connection conductor (22a; 22b) can be controlled during operation of the fluid warmer (10) by the control device (32) to activate/deactivate the resistance heating element (18).

11. Fluid warmer according to claim 1, **characterized in that** the compensation device (46) comprises an LC resonant circuit (48) with an inductor (L) and a capacitor (C).

12. Fluid warmer according to claim 11, **characterized in that** the inductor (L) of the LC resonant circuit (48) is constituted as a secondary side of a transformer (T), whose primary side is connected to the supply connection conductors (22a, 22b) of the resistance heating element (18).

13. Fluid warmer according to any one of the previous claims, **characterized in that** the heat transfer element (20) has a holding chamber (28) for a bag-shaped vessel (12a) of the fluid tube (12).

14. Fluid warmer according to any one of the previous claims, **characterized by** two or more resistance heating elements (18).

15. Fluid warmer according to any one of the previous claims, **characterized by** a power output totaling at least 0.15 KW, preferably more than 0.2KW.

16. Method (100) of operating a fluid warmer (10) for temperature control of a fluid (14) flowing in a fluid tube (12), comprising:

- a resistance heating element (18), with a first and a second electrical supply connection conductor (22a; 22b) for connecting the resistance heating element (18) to an alternating voltage source (24),
- a heat transfer element (20), which is thermally coupled to the resistance heating element (18), for transferring the heat to the fluid (14) flowing in a vessel (12a) of the fluid tube (12),

- a functional grounding conductor (FE) connected to the heat transfer element (20) for functional grounding of the heat transfer element (20);
- a sensor (38) for sensing the present leakage current 40 via the functional grounding conductor (FE);
- an electric or electronic compensation device (46) for compensation of the leakage current (40) induced by the resistance heating element (18); and
- a control device (32) for controlling switches (30a; 30b; 30c), with each of which the supply connection conductors (22a; 22b) and the functional grounding conductor (FE) are connected,

having the following steps:

- definition of a maximum threshold current magnitude ($I_{Ton}$, $I_{Toff}$) for a leakage current (40) via the functional grounding conductor (FE);
- at least partial compensation of the leakage current induced in the heat transfer element by the resistance heating element by means of the compensation device;
- comparison of the leakage current (40) measured with the sensor (38) with the defined maximum threshold current magnitude ($I_{Ton}$, $I_{Toff}$); and
- putting the switches (30a; 30b; 30c) into their open switching state within a defined time interval on occurrence of a leakage current (40), which is greater than or equal to the defined maximum threshold current magnitude ($I_{Ton}$, $I_{Toff}$).

17. Method according to claim 16, **characterized by** the following further steps:

- comparison of the leakage current (40) measured with the sensor (38) with a defined minimum threshold current magnitude ($I_{Lon}$, $I_{Loff}$); and
- putting the switches (30a; 30b; 30c) into their open switching state within the defined time interval on occurrence of a leakage current (40), which is less than or equal to the minimum threshold current magnitude ($I_{Lon}$, $I_{Loff}$).

**Revendications**

1. Réchauffeur de fluide (10) pour tempérer un fluide médical (14) guidé dans une conduite de fluide (12), avec

- un élément chauffant à résistance (18) pour tension alternative, qui présente un premier et un deuxième conducteur de raccordement au secteur électrique (22a, 22b) ; avec
- un échangeur de chaleur (20) qui est couplé thermiquement avec l'élément chauffant à résistance (18),
- un conducteur de mise à la terre fonctionnelle (FE) qui est relié de manière électroconductrice à l'échangeur de chaleur (20), avec
- un capteur (38) pour déterminer une intensité de courant ($I_L$) respective d'un courant de fuite (40) via le conducteur de mise à la terre fonctionnelle (FE),
- avec un dispositif de compensation (46) électrique ou électronique pour compenser le courant de fuite (40) induit par l'élément chauffant à résistance (18) et avec
- un dispositif de commande (32),

les deux conducteurs de raccordement au secteur (22a ; 22b) et le conducteur de mise à la terre fonctionnelle (FE) étant munis respectivement de commutateurs (30a ; 30b ; 30c), et

les commutateurs (30a ; 30b ; 30c) pouvant être passés ensemble dans leur état de commutation ouvert au moyen du dispositif de commande (32), dans un intervalle de temps prédéfini, lorsqu'un courant de fuite (40) est supérieur ou égal à une intensité de courant seuil maximale définie ($I_{TOn}$, $I_{TOff}$).

2. Réchauffeur de fluide selon la revendication 1, **caractérisé en ce que** des intensités de courant seuil ($I_{TOn}$, $I_{TOff}$) différentes sont prédéfinies pour un état de fonctionnement du réchauffeur de fluide (10) avec élément chauffant à résistance (18) activé et pour un état de fonctionnement avec élément chauffant à résistance (18) désactivé.

3. Réchauffeur de fluide selon l'une des revendications précédentes, **caractérisé en ce que** l'intensité de courant seuil maximale ($I_{Ton}$) correspond à la somme d'une intensité de courant ($I_{LOn}$) du courant de fuite (40) à une puissance de chauffage maximale de l'élément chauffant à résistance (18) et d'une intensité de courant différentielle ($\Delta I$) pouvant de préférence être prédéfinie librement.

4. Réchauffeur de fluide selon l'une des revendications précédentes, **caractérisé en ce que** l'intensité de courant seuil maximale ($I_{TOff}$) correspond à l'intensité de courant différentielle ($\Delta I$) ou à la somme de l'intensité de courant

différentielle (∆I) et d'une intensité de courant (I$_{LOff}$) du courant de fuite (40) lorsque le commutateur (30a ; 30b ; 30c) du conducteur de raccordement au secteur (22a, 22b) raccordé côté conducteur extérieur à une source de tension alternative (24) est fermé et le commutateur (30a ; 30b ; 30c) du conducteur de raccordement au secteur (22a; 22b) de l'élément chauffant à résistance (18) raccordé côté conducteur neutre à la source de tension alternative (24) est ouvert.

5. Réchauffeur de fluide selon l'une des revendications 3 ou 4, **caractérisé en ce que** l'intensité de courant différentielle (∆I) est comprise entre 1 microampère et 50 microampères au maximum.

6. Réchauffeur de fluide selon l'une des revendications précédentes, **caractérisé en ce que** les commutateurs (30a ; 30b ; 30c) peuvent être passés ensemble dans leur état de commutation ouvert dans l'intervalle de temps prédéfini lorsqu'un courant de fuite (40) est inférieur ou égal à une intensité de courant seuil minimale (I$_{LOn}$, I$_{LOff}$) prédéfinie.

7. Réchauffeur de fluide selon la revendication 6, **caractérisé en ce que** des intensités de courant seuil (I$_{TOn}$, I$_{TOff}$) différentes sont prédéfinies pour un état de fonctionnement du réchauffeur de fluide (10) avec élément chauffant à résistance (18) activé et pour un état de fonctionnement avec élément chauffant à résistance (18) désactivé.

8. Réchauffeur de fluide selon l'une des revendications précédentes, **caractérisé en ce que** l'intervalle de temps prédéfini, dans lequel les commutateurs (30a ; 30b ; 30c) peuvent être passés dans l'état de commutation ouvert par le dispositif de commande (32), est plus petit qu'une période, de préférence plus petit qu'une demi-période de la tension alternative utilisée pour le fonctionnement de l'élément chauffant à résistance (18).

9. Réchauffeur de fluide selon l'une des revendications précédentes, **caractérisé en ce que** les commutateurs (30a ; 30b ; 30c) sont réalisés sous la forme d'un thyristor diode bidirectionnel (TRIAC), d'un transistor à effet de champ à grille métal-oxyde (MOSFET) ou d'un relais et/ou que les commutateurs sont chaque fois réalisés en double.

10. Réchauffeur de fluide selon l'une des revendications précédentes, **caractérisé en ce que** le conducteur de raccordement au secteur (22a ; 22b) qui est raccordé de manière conductrice côté conducteur extérieur à une source de tension alternative (24) peut être identifié par le dispositif de commande (32) et que le commutateur (30a ; 30b ; 30c) de ce conducteur de raccordement au secteur (22a ; 22b) peut être commandé pendant le fonctionnement du réchauffeur de fluide (10) par le dispositif de commande (32) pour activer/désactiver l'élément chauffant à résistance (18).

11. Réchauffeur de fluide selon la revendication 1, **caractérisé en ce que** le dispositif de compensation (46) comprend un circuit oscillant LC (48) avec une bobine (L) et un condensateur (C).

12. Réchauffeur de fluide selon la revendication 11, **caractérisé en ce que** la bobine (L) du circuit oscillant LC (48) est configurée comme côté secondaire d'un transformateur (T) qui est raccordé côté primaire aux conducteurs de raccordement au secteur (22a, 22b) de l'élément chauffant à résistance (18).

13. Réchauffeur de fluide selon l'une des revendications précédentes, **caractérisé en ce que** l'échangeur de chaleur (20) présente une chambre de réception (28) pour un récipient (12a) en forme de sac de la conduite de fluide (12).

14. Réchauffeur de fluide selon l'une des revendications précédentes, **caractérisé par** deux ou plusieurs éléments chauffants à résistance (18).

15. Réchauffeur de fluide selon l'une des revendications précédentes, **caractérisé par** une puissance de sortie d'au moins 0,15 kW, de préférence supérieure à 0,2 kW.

16. Procédé (100) de fonctionnement d'un réchauffeur de fluide (10) pour tempérer un fluide (14) passant dans une conduite de fluide (12), comprenant :

- un élément chauffant à résistance (18), avec un premier et un deuxième conducteur de raccordement au secteur électrique (22a ; 22b) pour raccorder l'élément chauffant à résistance (18) à une source de tension alternative (24),
- un échangeur de chaleur (20) qui est couplé thermiquement avec l'élément chauffant à résistance (18), pour transmettre de la chaleur au fluide (14) qui passe dans un récipient (12a) de la conduite de fluide (12),
- un conducteur de mise à la terre fonctionnelle (FE) relié à l'échangeur de chaleur (20) pour la mise à la terre fonctionnelle de l'échangeur de chaleur (20) ;

- un capteur (38) pour détecter un courant de fuite 40 respectif via le conducteur de mise à la terre fonctionnelle (FE),

- un dispositif de compensation (46) électrique ou électronique pour compenser le courant de fuite (40) induit par l'élément chauffant à résistance (18) et

- un dispositif de commande (32) pour commander des commutateurs (30a ;

30b ; 30c) auxquels les conducteurs de raccordement au secteur (22a ; 22b) et le conducteur de mise à la terre fonctionnelle (FE) sont respectivement reliés, présentant les étapes suivantes :

- prédéfinition d'une intensité de courant seuil maximale ($I_{TOn}$, $I_{TOff}$) pour un courant de fuite (40) via le conducteur de mise à la terre fonctionnelle (FE) ;

- compensation au moins partielle du courant de fuite induit dans l'échangeur de chaleur par l'élément chauffant à résistance au moyen du dispositif de compensation ;

- comparaison du courant de fuite (40) détecté avec le capteur (38) avec l'intensité de courant seuil maximale ($I_{TOn}$, $I_{TOff}$) prédéfinie ; et

- passage des commutateurs (30a ; 30b ; 30c) dans leur état de commutation ouvert dans un intervalle de temps prédéfini lorsqu'un courant de fuite (40) est supérieur ou égal à l'intensité de courant seuil maximale ($I_{TOn}$, $I_{TOff}$) prédéfinie.

17.  Procédé selon la revendication 16, **caractérisé par** les étapes supplémentaires suivantes :

- comparaison du courant de fuite (40) détecté avec le capteur (38) avec une intensité de courant seuil minimale ($I_{LOn}$, $I_{LOff}$) prédéfinie ; et

- passage des commutateurs (30a ; 30b ; 30c) dans leur état de commutation ouvert dans l'intervalle de temps prédéfini lorsqu'un courant de fuite (40) est inférieur ou égal à l'intensité de courant seuil minimale ($I_{LOn}$, $I_{LOff}$).

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010036295 A1
- EP 0504835 A2